# EUROPÄISCHE PATENTSCHRIFT

(11) **EP 1 552 907 B1**
(45) Veröffentlichungstag und Bekanntmachung des Hinweises auf die Patenterteilung: **10.01.2007**
(21) Anmeldenummer: 05000671.7
(22) Anmeldetag: 31.10.2000
(51) Int. Cl.: B25B 33/00

(54) **Gerät zum Öffnen und Schliessen einer Vorrichtung zur Verabreichung von Flüssigkeiten**
Apparatus for opening and closing of a device for administering liquids
Appareil pour l'ouverture et la fermeture d'un dispositif d'administration de liquides

(43) Veröffentlichungstag der Anmeldung: 13.07.2005
(62) Teilanmeldung aus: 00988555.9
(73) Patentinhaber: Onkoworks Gesellschaft für Herstellung und Vertrieb onkologischer Spezialpräparate mbH, 42781 Haan (DE)
(72) Erfinder: Fischer, Wilfried, 42781 Haan (DE); Brücker, Matthias, 9200 Gossau (CH)
(74) Vertreter: Hauck, Graalfs, Wehnert, Döring, Siemons

(56) Entgegenhaltungen:
- FR-A- 2 569 675

## Beschreibung

Die vorliegende Erfindung betrifft ein Gerät zum Öffnen und Schließen einer Vorrichtung zur Verabreichung von Flüssigkeiten, insbesondere Infusionslösungen, mit
einer unteren Gehäusehälfte 2,
einer schwenkbar mit der unteren Gehäusehälfte 2 verbundenen oberen Gehäusehälfte 1,
einem die Gehäusehälften 1, 2 im geschlossenen Zustand zur Ausbildung eines Gehäuses zusammenhaltenden Verschluss mit an der oberen Gehäusehälfte 1 angreifenden Verschlusslaschen 8,
einem im Gehäuse angeordneten Druckstempel 3 zur Beaufschlagung eines zwischen die untere Gehäusehälfte 2 und den Druckstempel 3 gelegten, mit der zu verabreichenden Flüssigkeit gefüllten Beutels 15 und
einem dem Druckstempel 3 bei geschlossenem Gehäuse und eingelegtem Beutel 15 mit einer solchen Kraft beaufschlagenden Mechanismus, dass ein Druck im Beutel 15 erzeugt wird, der die Abgabe eines konstanten Flüssigkeitsvolumens pro Zeiteinheit über den Zeitraum des Verabreichungsprozesses bewirkt.

Eine Vorrichtung der vorstehend beschriebenen Art ist in der WO 02/36185 A1 offenbart. Diese Vorrichtung zeichnet sich dadurch aus, dass sie ohne Zuhilfenahme von Spezialwerkzeug nicht öffenbar ist. Hierdurch soll verhindert werden, dass unerwünschte Manipulationen von Laien, beispielsweise vom Patienten selbst, durchgeführt werden können. Der Verschluss ist daher so ausgebildet, dass ein Öffnen des Gehäuses mit den Fingern nicht möglich ist.

Die Druckschrift FR-A-2 569 675 offenbart ein Gerät zum Öffnen von sterilisierten Haushaltsgläsern, wobei das Gerät zwei gegeneinander verschwenkbare Arme aufweist, von denen eines eine Öffnungseinrichtung in Form eines Querbalkens mit Pressabschnitt besitzt.

Der vorliegenden Erfindung liegt die Aufgabe zugrunde, ein Gerät zum Öffnen und Schließen einer derartigen Vorrichtung zu schaffen, das in einfacher und bequemer Weise ein Öffnen und Schließen einer derartigen Vorrichtung ermöglicht.

Diese Aufgabe wird erfindungsgemäß durch ein Gerät gelöst, das einen unteren stationären Lagerteil zur Fixierung der Vorrichtung und einen manuell handhabbaren, bügelartigen oberen Teil, der mit dem unteren Lagerteil schwenkbar verbunden ist, einen zwischen seinen Längsschenkeln drehbeweglich angeordneten Querabschnitt zum Pressen gegen die obere Gehäusehälfte und somit zum Schließen der Vorrichtung und eine Öffnungseinrichtung in Form eines Querbalkens mit Pressabschnitt sowie mit in den beiden seitlichen Endbereichen des Querbalkens angeordneten keilförmigen Abschnitten zum Pressen der an der oberen Gehäusehälfte der Vorrichtung angreifenden Verschlusslaschen seitlich nach außen und damit zum Lösen der Verrastung zwischen beiden Gehäusehälften besitzt, aufweist.

Das erfindungsgemäße Gerät zum Schließen und Öffnen (Ladegerät) funktioniert so, dass die leere Vorrichtung geöffnet in das Gerät eingesetzt wird. Der gefüllte Beutel wird in die untere Gehäusehälfte der Vorrichtung eingelegt. Mit dem bügelartigen oberen Teil kann die obere Gehäusehälfte der Vorrichtung mit einem vernünftigen Kraftaufwand gegen die untere Gehäusehälfte gepresst werden, bis der Verschluss einrastet. Die Vorrichtung ist dann betriebsbereit.

Zum Öffnen der Vorrichtung werden durch manuelles Niederdrücken des Pressabschnittes die keilförmigen Abschnitte nach unten bewegt, wodurch die Verschlusslaschen seitlich nach außen bewegt und von der oberen Gehäusehälfte gelöst werden. Die obere Gehäusehälfte schnappt dann nach oben, so dass die Vorrichtung dann offen ist. Sie kann aus dem Ladegerät entfernt und wieder mit einem neuen gefüllten Beutel beschickt werden.

Der untere stationäre Lagerteil ist vorzugsweise rahmenförmig ausgebildet.

Ferner besitzt der untere stationäre Lagerteil zweckmäßigerweise einen ausziehbaren Teil mit einem vorderen Griff. Der bügelartige obere Teil besitzt vorzugsweise ebenfalls einen ausziehbaren Teil, an dessen Vorderseite ein Handgriff angeordnet ist. Auf diese Weise können der untere und der obere Teil auf eine geeignete Länge gebracht und die Laschen ohne viel Kraftaufwand auf die Seite geschoben werden.

Die Erfindung wird nachfolgend anhand eines Ausführungsbeispieles in Verbindung mit der Zeichnung im einzelnen erläutert. Es zeigen:
- Figur 1: eine räumliche Ansicht einer Vorrichtung zur Verabreichung von Flüssigkeiten im geöffneten Zustand, wobei die Federn, Zugseile und Lagereinrichtungen für die Kipphebel im von der oberen Gehäusehälfte gelösten Zustand dargestellt sind; und
- Figur 2: eine räumliche Darstellung eines Gerätes zum Öffnen und Schließen der Vorrichtung (Ladegerätes).

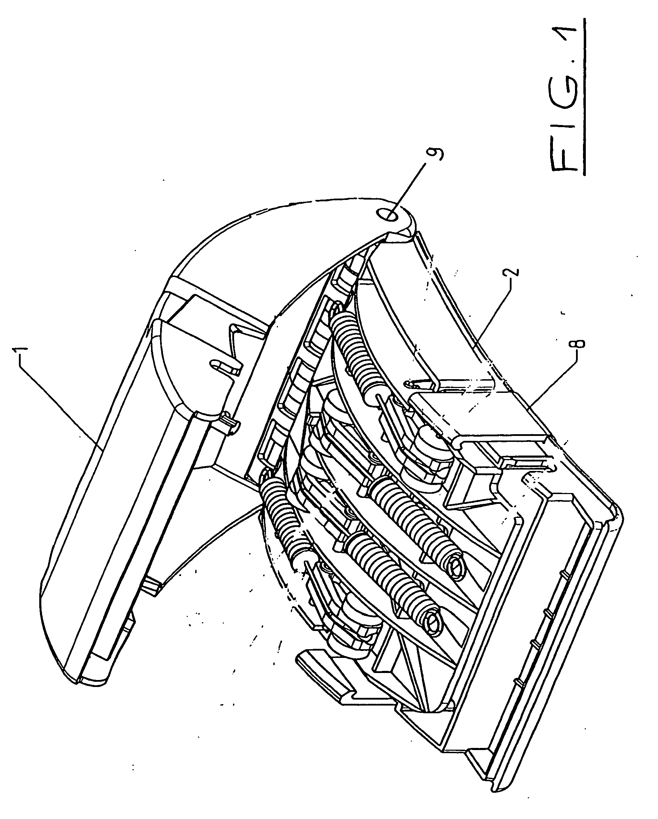

Wie man Figur 1 entnehmen kann, umfasst die dargestellte Vorrichtung zur Verabreichung von Flüssigkeiten, bei der es sich im vorliegenden Fall um eine Infusionspumpe handelt, eine obere Gehäusehälfte 1 und eine untere Gehäusehälfte 2, die über ein bei 9 gezeigtes Scharnier schwenkbar miteinander verbunden sind. Beide Hälften bilden im geschlossenen Zustand ein Gehäuse, das eine flache, etwa rechteckige Form besitzt und einer Druckschnalle nahe kommt. Auf der Rückseite der unteren Gehäusehälfte 2 sind zwei Gurtlaschen angeordnet, durch die ein Gurt gezogen werden kann, mit dem die Infusionspumpe am Körper getragen werden kann.

Wenn die oberen Gehäusehälfte 1 zum Schließen verschwenkt wird, werden zwei seitlich an der unteren Gehäusehälfte 2 angeordnete Verschlusslaschen 8 etwas nach außen gedrückt und gleiten in einem entsprechenden Bereich der oberen Gehäusehälfte 1 nach oben, bis sie schließlich in einen entsprechenden Freiraum nach innen schnappen und somit beide Gehäusehälften fest miteinander verriegeln. Die Gehäusehälften lassen sich dann nicht mehr ohne weiteres öffnen, vielmehr wird hierzu das nachfolgend beschriebene Spezialgerät benötigt.

Der Innenaufbau der Vorrichtung hat für die hier beschriebene Erfindung keine Bedeutung und wird daher nicht weiter erläutert. Einzelheiten hiervon sind in der bereits vorstehend genannten WO 02/36185 A1 beschrieben.

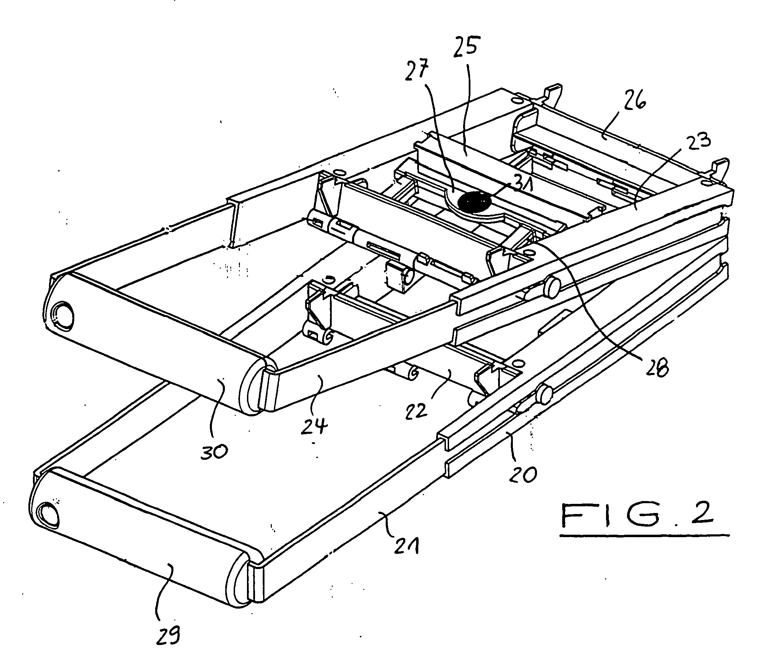

Figur 2 zeigt ein Gerät zum Öffnen und Schließen der in Figur 1 gezeigten Vorrichtung. Das Gerät besitzt einen rahmenförmigen unteren Teil 20, der einen ausziehbaren Teil 21 mit einem vorderen Griff 29 besitzt. Der rahmenförmige untere Teil 20 bildet ein Lager für die zu öffnende oder zu schließende Vorrichtung, die dort zwischen den beiden Längsschenkeln sowie dem vorderen Querschenkel 22 und dem hinteren Querschenkel des rahmenförmigen Unterteiles 20 fixiert werden kann.

Der untere Lagerteil 20 ist schwenkbar mit einem oberen Teil 23 verbunden, der bügelartig ausgebildet ist und ebenfalls einen ausziehbaren Teil 24 aufweist, an dessen Vorderseite ein Handgriff 30 angeordnet ist. Mit Hilfe der Griffe 29 und 30 können die beiden Teile 20 und 23 auf eine geeignete Länge gebracht werden. Das bügelartige obere Teil 23 wird nach dem Einlegen der zu schließenden Vorrichtung nach unten bewegt, wobei ein zwischen den Längsschenkeln des Teiles 23 angeordneter Querabschnitt 25, der drehbeweglich angeordnet ist, gegen die Oberseite der oberen Gehäusehälfte der Vorrichtung gepresst wird. Auf diese Weise wird die Vorrichtung geschlossen.

Zwischen den Längsschenkeln des Oberteiles 23 ist ferner eine Öffnungseinrichtung 27, d.h. eine Einrichtung zum Lösen der Verrastung der Verschlusslaschen mit dem anderen Gehäuseteil der Vorrichtung vorgesehen. Diese Öffnungseinrichtung 27 hat einen Querbalken mit einem bei 31 angedeuteten Pressabschnitt, an dem mittels Fingerdruck der Querbalken nach unten bewegt wird. In den beiden seitlichen Endbereichen des Querbalkens sind keilförmige Abschnitte 28 angeordnet, die bei der Abwärtsbewegung die an der oberen Gehäusehälfte der zu öffnenden Vorrichtung angreifenden Verschlusslaschen seitlich nach außen pressen, so dass die Verrastung zwischen beiden Gehäusehälften gelöste wird. Hierdurch schnappt die obere Gehäusehälfte der Vorrichtung etwas nach oben und kann somit manuell geöffnet werden. Die Vorrichtung kann dann aus dem Ladegerät entnommen werden, um den verbrauchten Beutel zu entfernen und einen neuen Beutel einzulegen.

Mit anderen Worten, die Verriegelung (Verschlusslaschen) ist manuell nicht lösbar, da durch den sehr hohen Federdruck im Inneren die Verschlusslasche so stark in die Kerbe am Gehäuseoberteil gepresst wird, dass die Verschlusslasche bei bestehendem Innendruck nur sehr schwer aus der Rastposition gelöst werden kann. Das Ladegerät dient nun dazu, mittels des Querabschnittes 25 und des ausziehbaren Hebels 24 diesem Innendruck entgegenzuwirken, damit die Verschlusslaschen aus der Rastposition freigegeben werden und so mit der Öffnungseinrichtung 27 die Laschen ohne viel Kraftaufwand auf die Seite geschoben werden können.

## Patentansprüche

1. Gerät zum Öffnen und Schließen einer Vorrichtung zur Verabreichung von Flüssigkeiten, insbesondere Infusionslösungen, mit
einer unteren Gehäusehälfte (2),
einer schwenkbar mit der unteren Gehäusehälfte (2) verbundenen oberen Gehäusehälfte (1),
einem die Gehäusehälften (1, 2) im geschlossenen Zustand zur Ausbildung eines Gehäuses zusammenhaltenden Verschluss mit an der oberen Gehäusehälfte (1) angreifenden Verschlusslaschen (8),
einem im Gehäuse angeordneten Druckstempel (3) zur Beaufschlagung eines zwischen die untere Gehäusehälfte (2) und den Druckstempel (3) gelegten, mit der zu verabreichenden Flüssigkeit gefüllten Beutels (15) und
einem dem Druckstempel (3) bei geschlossenem Gehäuse und eingelegtem Beutel (15) mit einer solchen Kraft beaufschlagenden Mechanismus, dass ein Druck im Beutel (15) erzeugt wird, der die Abgabe eines konstanten Flüssigkeitsvolumens pro Zeiteinheit über den Zeitraum des Verabreichungsprozesses bewirkt,
wobei das Gerät einen unteren stationären Lagerteil (20) zur Fixierung der Vorrichtung und einen manuell handhabbaren, bügelartigen oberen Teil (23), der mit dem unteren Lagerteil (20) schwenkbar verbunden ist, einen zwischen seinen Längsschenkeln drehbeweglich angeordneten Querabschnitt (25) zum Pressen gegen die obere Gehäusehälfte (1) und somit zum Schließen der Vorrichtung und eine Öffnungseinrichtung (27) in Form eines Querbalkens mit Pressabschnitt (31) sowie mit in den beiden seitlichen Endbereichen des Querbalkens angeordneten keilförmigen Abschnitten (28) zum Pressen der an der oberen Gehäusehälfte (1) der Vorrichtung angreifenden Verschlusslaschen (8) seitlich nach außen und damit zum Lösen der Verrastung zwischen beiden Gehäusehälften (1, 2) besitzt, aufweist.

2. Gerät nach Anspruch 1, **dadurch gekennzeichnet, dass** der untere stationäre Lagerteil (20) rahmenförmig ausgebildet ist.

3. Gerät nach Anspruch 1 oder 2, **dadurch gekennzeichnet, dass** der untere stationäre Lagerteil (20) einen ausziehbaren Teil (21) mit einem vorderen Griff (29) besitzt.

4. Gerät nach einem der vorangehenden Ansprüche, **dadurch gekennzeichnet, dass** der bügelartige obere Teil (23) einen ausziehbaren Teil (24) aufweist, an dessen Vorderseite ein Handgriff (30) angeordnet ist.

## Claims

1. An apparatus for opening and closing a device for administering liquids, especially infusion solutions, said device comprising
a lower casing half (2),
an upper casing half (1) connected to the lower casing half (2),
a closure holding together the casing halves (1, 2) in a closed condition for the formation of a casing, said closure having closing tongues (8) acting on the upper casing half (1),
a pressure piston (3) located within the casing for acting on a bag (15) filled with the liquid to be administered and laid between the lower casing half (2) and the pressure piston (3) and
a mechanism applying such a force to the pressure piston (3) when the bag (15) is laid in and the casing is closed that a pressure is generated in the bag (15) which causes the discharge of a constant liquid volume per unit of time over the period of time of the administering process,
wherein the apparatus includes a lower stationary support part (20) for the fixation of the device and a bow-like upper part (23) which can be manually handled and which is pivotally connected to the lower support part (20), a cross portion (25) rotationally located between its longitudinal legs for pressing against the upper casing half (1) and thus for closing the device, and opening means (27) in the shape of a crosspiece with a pressing portion (31) and with wedge-like portions (28) located in the two lateral end portions of the crosspiece for pressing the closing tongues (8) acting on the upper casing half (1) of the device laterally outwardly and thus for releasing the locking between the two casing halves (1, 2).

2. The apparatus according to claim 1, **characterized in that** the lower stationary support part (20) is formed in a frame-like manner.

3. The apparatus according to claim 1 or 2, **characterized in that** the lower stationary support part (20) has an extensible part (21) with a front grip (29).

4. The apparatus according to one of the preceding claims, **characterized in that** the bow-like upper part (23) has an extensible part (24) having a handle (30) located at its front side.

## Revendications

1. Appareil pour l'ouverture et la fermeture d'un dispositif d'administration de liquides, en particulier de solutions de perfusion, comprenant:
une moitié inférieure (2) de logement,
une moitié supérieure (1) de logement reliée de manière basculante à la moitié inférieure (2) de logement,
une fermeture maintenant les moitiés (1, 2) de logement à l'état fermé pour former un logement, comprenant une languette de fermeture (8) en prise avec la moitié supérieure (1) de logement,
un piston de compression (3) disposé dans le logement pour agir sur une poche (15) placée entre la moitié inférieure (2) de logement et le piston de compression (3), remplie du liquide à administrer, et
un mécanisme agissant sur le piston de compression (3) quand le logement est fermé et la poche (15) mise en place avec une telle force qu'une pression est engendrée dans la poche (15), pression, qui a pour effet la délivrance d'un volume constant de liquide par unité de temps au cours de la période de temps du processus d'administration,
l'appareil présentant une partie de support inférieure. (20) stationnaire pour fixer le dispositif et une partie supérieure (23) en forme d'arceau, à manipuler manuellement, qui est reliée de manière pivotante à la partie de support inférieure (20), une section transversale (25) disposée de manière mobile et rotative entre ses branches longitudinales pour presser contre la moitié supérieure (1) de logement et pour fermer ainsi le dispositif, et un mécanisme d'ouverture (27) en forme de traverse avec une section de pression (31) ainsi qu'avec des sections en forme de coin (28) disposées dans les deux zones d'extrémité latérale de la traverse pour presser latéralement vers l'extérieur les languettes de fermeture (8) en prise avec la moitié supérieure (1) de logement et débloquer ainsi l'encliquetage entre les deux moitiés (1, 2) de logement.

2. Appareil selon la revendication 1, **caractérisé en ce que** la partie de support inférieure (20) stationnaire est configurée en formé de cadre.

3. Appareil selon la revendication 1 ou 2, **caractérisé en ce que** la partie de support inférieure (20) stationnaire a une partie extensible (21) avec une poignée avant (29).

4. Appareil selon l'une des revendications précédentes, **caractérisé en ce que** la partie supérieure (23) en forme d'arceau présente une partie extensible (24) sur le côté avant de laquelle une poignée (30) est disposée.
